# EUROPEAN PATENT APPLICATION

(11) **EP 2 055 231 A1**
(43) Date of publication of application: **06.05.2009**
(21) Application number: 08075853.5
(22) Date of filing: 30.10.2008
(51) Int. Cl.: A61B 5/0408, A61B 5/0488, A61B 5/0492

(54) **Flexible electrode holder**

(30) Priority: 31.10.2007 NL 1034611
(71) Applicant: Roessingh Research & Development B.V., 7522 AH Enschede (NL)
(72) Inventor: De Nooij, Ruud, 6645 KA Winssen (NL); Scholten, Bianca Georgette, 7482 AZ Haaksbergen (NL); Hermens, Hermanus Jacobus, 7522 CD Enschede (NL); Kallenberg, Laura Anna Cornelia, 7512 DG Enschede (NL)
(74) Representative: 't Jong, Bastiaan Jacob

(57) **Abstract**

The invention relates to a device for holding electrodes against the skin, which device comprises:
- a holder frame; and
- at least two electrodes arranged pivotally on the holder frame.

The invention further relates to a combination of a device according to the invention and an adjustable belt to which the device is attached, and using which belt the device can be arranged around a part of the body, for instance the waist of a person.

## Description

The invention relates to a device for holding and holding in position electrodes, for instance EMG electrodes, against the skin, in particular the skin on the back muscles.

The number of people having chronic pain is high and has increased in recent years. It is normal to recover from neck complaints or back complaints within a period of time, but for a number of people these disorders become chronic.

Chronic pain is caused by multiple factors, such as physical characteristics of the individual, personal factors, nature of work, characteristics of the social environment as well as the interplay of these individual factors. A factor often identified in the case of chronic pain is abnormal tensioning of muscles as a response to pain. People change their muscle tension in response to pain in order to prevent further pain. This can take place in different ways. Some people avoid movement and attempt to spare painful muscles, while others make movements but stiffen the painful area. Abnormal muscle tension occurs in both cases.

Insight into normal and abnormal muscle tension can be obtained by measuring the muscle activity. The measurements take place by recording the electric signals caused by muscle activity using electrodes placed on the skin above the muscle. Because many factors play a part in the cause and persistence of chronic pain, it is difficult in a laboratory environment to gain a good insight into muscle activity. Work-related factors and social environment are for instance difficult to simulate. It is therefore desirable to also be able to perform measurements on the muscles during everyday activities. It is of great importance here that the electrodes are arranged in each case at exactly the same location on the skin and also that they remain at this location during the diverse movements of the user. A second requirement is that the user is not hindered in his/her daily activities. Finally, it is important that the electrodes can be arranged and removed independently, so without the assistance of third parties.

This object is achieved with a device according to the invention, which device comprises:
- a holder frame; and
- at least two electrodes arranged pivotally on the holder frame.

Using such a device it is easy to arrange two electrodes at a fixed distance from each other on the skin in order to thus measure the electrical activity of a muscle. Because the electrodes are arranged pivotally, they can adapt dynamically to the curves of the skin. Through the use of a holder frame the distance between the two electrodes is at the same time kept constant, this being of great importance because the characteristics of the EMG greatly depend on the inter-electrode distance. This prevents the distance between the electrodes varying considerably due to stretching of the skin, as is the case with for instance self-adhesive electrodes. The holder frame moreover has the advantage that the device, and thereby the electrodes, can easily be removed and rearranged. This also in contrast to for instance self-adhesive electrodes, which are adhered fixedly to the skin and are therefore difficult to remove.

In another preferred embodiment of the device according to the invention each holder frame is provided with electrode holders pivotable around a shaft arranged in the holder frame. This ensures that the holder frames can pivot only in a single dimension in accordance with the curving of the relevant muscle. The electrodes are here preferably arranged releasably in the electrode holders. This simplifies exchange of the electrodes when they must for instance be replaced.

The invention further comprises a device comprising:
- a flexible frame element; and
- devices according to the invention arranged on either side of the flexible frame element.

The flexible frame element and the pivotally arranged electrodes ensure that the electrodes are accommodated in a device, whereby the distance can be preset, while the electrodes, because they are pivotable, can keep contact with the skin at all times, also during movement.

As electrodes can be used both self-adhesive electrodes, which make contact via a gel, or dry electrodes which make contact with the skin as a result of natural transpiration.

In a preferred embodiment of the device according to the invention the flexible frame element is a leaf spring. Using this leaf spring it is easy to place the two holder frames at the correct angle relative to each other. This is advantageous in the case of very curved skin parts, such as for instance the skin of the upper leg or a back.

The leaf spring can further be curved such that the outer ends of the leaf spring are at an angle in longitudinal direction. This is important particularly for measurements on the back muscles lying on either side of the spinal column. The shape of the back can differ per patient. It is thus possible for the back muscles to be highly developed, whereby the spinal column lies deeper than the back muscles, while the opposite is also possible.

In another embodiment of a device according to the invention the holder frames are arranged slidably on the frame element such that the mutual distance between the holder frames is adjustable. It hereby becomes possible to position the electrodes in the holders properly relative to the muscles.

The invention also comprises a combination of a device according to the invention and an adjustable belt to which the device is attached, and using which belt the device can be arranged around a part of the body, for instance the waist of a person. With such a combination the use of a leaf spring is preferred because this prevents the belt changing the relative position of the two holder frames and impeding good contact of the electrodes with the skin.

In a further embodiment of the combination according to the invention braces are arranged on the belt. This prevents the belt slipping downward and the device shifting relative to the muscles.

These and other features of the invention are further elucidated with reference to the accompanying drawings.
Figure 1 shows schematically a person wearing a combination according to the invention.
Figure 2 shows a perspective view of the device of the combination according to figure 1.
Figures 3 and 4 show a side view of the device of figure 2 in two different positions.

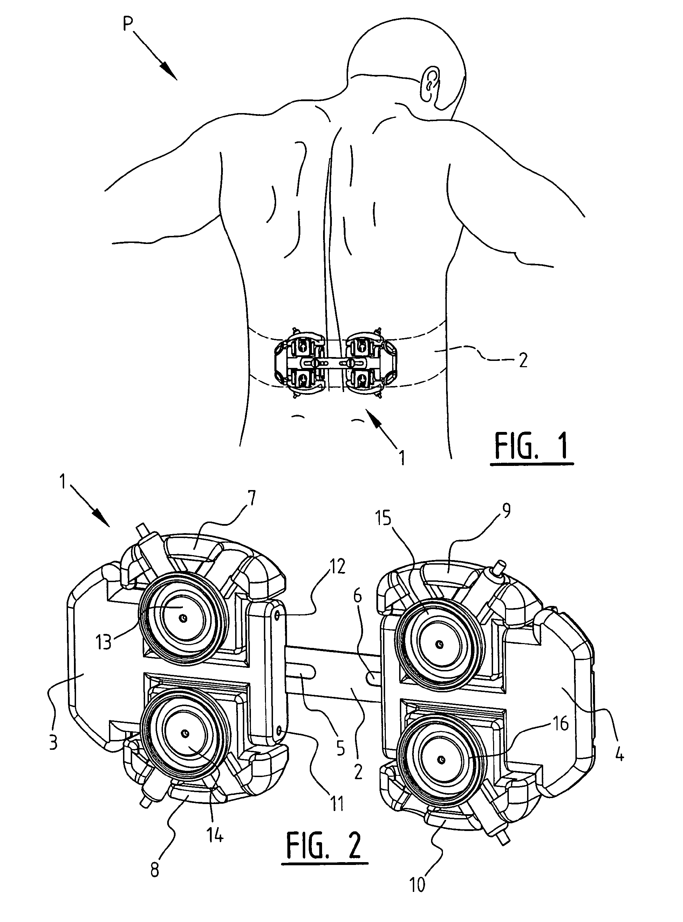

Figure 1 shows a person P wearing a device 1 by means of an adjustable belt 2. This adjustable belt 2 runs over device 1 so that the latter is situated between belt 2 and person P.

Figure 2 shows a perspective view of device 1. Device 1 has a flexible leaf spring 2, on the two outer ends of which a holder frame 3,4 is arranged. This holder frame 3,4 can be adjusted on leaf spring 2 by means of slots 5,6 respectively in order to set the mutual distance between holder frames 3,4. Each holder frame 3,4 has two electrode holders 7,8,9,10 which are arranged pivotally on holder frame 3, 4 by means of a shaft 11, 12. Provided in electrode holders 7,8,9,10 are recesses in which electrodes 13, 14, 15, 16 are snapped fixedly. The cables of these electrodes can here be provided with a pull relief, for instance a loop, connected to the device.

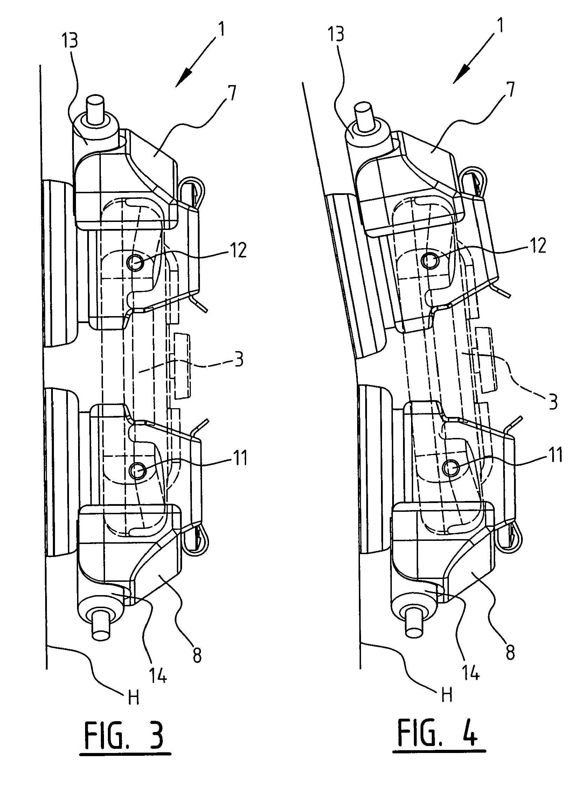

Figure 3 shows device 1 arranged on a skin surface H. Electrodes 13,14 here lie at a fixed distance, as is usual when performing measurements. Electrodes 13,14 are arranged in electrode holders 7, 8 which are in turn arranged in holder frame 3 via pivot shafts 11,12.

When the skin H now bends as shown in figure 4, electrodes 13,14 will continue to maintain contact with the skin H because they can pivot relative to each other around pivot shafts 11, 12. This ensures that, irrespective of the movement of the skin H, a good contact is maintained at all times and it is possible to continue performing the measurements.

## Claims

1. Device for holding electrodes against the skin, which device comprises:
- a holder frame; and
- at least two electrodes arranged pivotally on the holder frame.

2. Device as claimed in claim 1, wherein the holder frame is provided with electrode holders pivotable around a shaft arranged in the holder frame.

3. Device as claimed in claim 2, wherein the electrodes are arranged releasably in the electrode holders.

4. Device for holding electrodes against the skin, in particular the skin on the back muscles, which device comprises:
- a flexible frame element; and
- devices as claimed in any of the foregoing claims arranged on either side of the flexible frame element.

5. Device as claimed in claim 4, wherein the flexible frame element is a leaf spring.

6. Device as claimed in claim 5, wherein the leaf spring is curved such that the outer ends of the leaf spring are at an angle in longitudinal direction.

7. Device as claimed in any of the foregoing claims 4-6, wherein the holder frames are arranged slidably on the frame element such that the mutual distance between the holder frames is adjustable.

8. Combination of a device as claimed in any of the foregoing claims and an adjustable belt to which the device is attached, and using which belt the device can be arranged around a part of the body, for instance the waist of a person.

9. Combination as claimed in claim 8, wherein braces are arranged on the belt.
